(19) Europäisches Patentamt
European Patent Office
Office européen des brevets

(11) **EP 3 601 221 B1**

(12) **FASCICULE DE BREVET EUROPEEN**

(45) Date de publication et mention de la délivrance du brevet:
**05.05.2021 Bulletin 2021/18**

(21) Numéro de dépôt: **18714332.6**

(22) Date de dépôt: **19.03.2018**

(51) Int Cl.:
*C07C 309/15* *(2006.01)*

(86) Numéro de dépôt international:
**PCT/FR2018/050660**

(87) Numéro de publication internationale:
**WO 2018/172683 (27.09.2018 Gazette 2018/39)**

(54) **PROCEDE POUR LE TRAITEMENT D'UNE SUSPENSION DE PARTICULES SOLIDES DANS L'EAU UTILISANT UN (CO)POLYMERE D'UNE FORME CRISTALLINE HYDRATEE DE L'ACIDE 2-ACRYLAMIDO-2-METHYLPROPANE SULFONIQUE**

VERFAHREN ZUR BEHANDLUNG EINER SUSPENSION FESTER PARTIKEL IN WASSER UNTER VERWENDUNG EINES (CO)POLYMERS AUS EINER HYDRATISIERTEN KRISTALLINEN FORM VON 2-ACRYLAMIDO-2-METHYLPROPANSULFONSÄURE

METHOD FOR TREATING A SUSPENSION OF SOLID PARTICLES IN WATER USING A (CO)POLYMER OF A HYDRATED CRYSTALLINE FORM OF 2-ACRYLAMIDO-2-METHYLPROPANESULFONIC ACID

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.03.2017 FR 1752288**

(43) Date de publication de la demande:
**05.02.2020 Bulletin 2020/06**

(73) Titulaire: **SPCM SA**
**42160 Andrézieux-Bouthéon (FR)**

(72) Inventeurs:
• **FAVERO, Cédrick**
**42160 Andrezieux Boutheon (FR)**

• **KIEFFER, Johann**
**42160 Andrezieux Boutheon (FR)**
• **DAGUERRE, Frédéric**
**42160 Andrezieux Boutheon (FR)**

(74) Mandataire: **Cabinet Laurent & Charras**
**Le Contemporain**
**50 Chemin de la Bruyère**
**69574 Dardilly Cedex (FR)**

(56) Documents cités:
**WO-A1-2016/128638    US-A1- 2010 274 048**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

**DOMAINE DE L'INVENTION**

La présente invention concerne un procédé pour le traitement d'une suspension de particules solides dans l'eau, telle que des résidus de l'industrie minière, utilisant des (co)polymères hydrosolubles préparés à partir de la forme cristalline hydratée de l'acide 2-acrylamido-2-méthylpropane sulfonique ou d'au moins un de ses sels. Ce procédé comprend l'étape de mise en contact de ladite suspension avec des (co)polymères hydrosolubles obtenus à partir de la forme cristalline hydratée de l'acide 2-acrylamido-2-méthylpropane sulfonique ou d'au moins un de ses sels.

Le procédé selon l'invention consiste, entre autres, à ajouter lesdits (co)polymères hydrosolubles dans un épaississeur contenant cette suspension à traiter et/ou pendant le transport de ladite suspension vers une zone de dépôt pour sa déshydratation et sa solidification et/ou à ajouter lesdits (co)polymères à ladite suspension puis à effectuer un traitement mécanique, tel qu'une centrifugation, un pressage ou une filtration.

**ETAT ANTERIEUR DE LA TECHNIQUE**

Les suspensions de particules solides dans l'eau comprennent tous les types de boues, de résidus ou de matériaux de rebut. Les suspensions peuvent résulter de la transformation de minerais. Il s'agit par exemple de boues ou de résidus industriels et de tous les produits de lavage et de rebut de mines résultant de l'exploitation minière, comme par exemple les mines de charbon, les mines de diamant, les mines de phosphate, les mines de métal (aluminium, platine, fer, or, cuivre, argent, etc....). Les suspensions peuvent également résulter de boues ou de résidus d'extraction dérivés du traitement de sable bitumineux. Ces suspensions de particules solides comprennent généralement des particules organiques et/ou minérales, telles que par exemple des argiles, des sédiments, du sable, des oxydes métalliques, du pétrole, etc.... mélangées avec de l'eau.

Le terme « suspension » est utilisé par la suite et se réfère aux suspensions de particules solides telles que décrites ci-dessus.

Le traitement de ces résidus et d'autres matériaux de rebut est devenu un problème technique, environnemental et d'ordre public. L'utilisation de polymères synthétiques ou naturels, tels que des coagulants et des floculants, pour séparer les solides du liquide est une pratique courante.

Pendant longtemps, et même de nos jours, la boue minérale produite par des procédés de traitement physiques ou chimiques de minerais était stockée à ciel ouvert dans des bassins, des étangs, des barrages de rétention ou des remblais sous forme semi-liquide. Ces grands volumes de boue stockée créent par conséquent un véritable danger, en particulier si les digues lâchent.

Puisque les solutions de stockage traditionnelles sont manifestement dangereuses, de plus en plus de réglementations nationales ont été publiées, interdisant l'abandon de ces zones. Les réglementations appellent également à une obligation de remise en état de ces sites, c'est-à-dire le traitement et la consolidation des sols.

L'amélioration des traitements chimiques et mécaniques de résidus ou de boues est par conséquent un défi important.

Différentes tentatives ont été effectuées au cours des dernières décennies pour augmenter la vitesse de sédimentation des résidus afin de recycler de manière efficace l'eau et de réduire le volume des résidus. Les traitements physiques principaux comprennent la centrifugation, la filtration, l'électrophorèse et l'électrocoagulation.

D'autre part, des procédés chimiques ont émergés. Ils comprennent un procédé impliquant l'addition de produits chimiques, tels que le silicate de sodium, des floculants organiques, des coagulants inorganiques, des agents d'oxydation et de réduction et, plus récemment, le dioxyde de carbone.

En 1979-1980, Alsthom Atlantique et SNF (brevet US 4 347 140) ont mis au point un système de floculation à étapes multiples (superfloculation) spécifiquement conçu pour traiter des bassins de résidus d'argile provenant de la production de phosphates en Floride.

Le traitement de suspension a été étudié de manière continue : en 1986 selon le procédé décrit dans le document CA 1 273 888, puis en 1994 dans le document WO 96/05146, en 2000 dans le document CA 2 407 869 et en 2004 dans le document CA 1 515 581.

Dans le document CA 2 682 542, le procédé implique l'addition de polymères modifiés par copolymérisation et/ou ramification. Les polymères dotés de groupes hydrophobes, qui ont également été étudiés, ont montré une amélioration du traitement des suspensions. Le document WO2016/128638 décrit un procédé de traitement d'une suspension aqueuse de particules solides en utilisant polymères solubles comprenant des monomères comme par exemple l'acide 2-acrylamido-2-méthylpropane sulfonique.

Malgré de grandes avancées au cours des dernières années, il existe toujours un besoin de mise au point de (co)polymères qui permettent d'augmenter la vitesse et la quantité d'eau libérée à partir des suspensions. L'amélioration des caractéristiques physiques de la boue déshydratée produite est également recherchée.

**EXPOSE DE L'INVENTION**

La présente invention répond aux besoins ci-dessus grâce à un procédé de traitement de suspensions de particules solides dans l'eau utilisant au moins un (co)polymère hydrosoluble préparé à partir de la forme cristalline hydratée de l'acide 2-acrylamido-2-méthylpropane sulfonique ou d'au moins un de ses sels.

L'invention concerne un procédé de traitement d'une suspension de particules solides dans l'eau, comprenant la mise en contact de ladite suspension avec au moins un (co)polymère hydrosoluble préparé à partir de la forme cristalline hydratée de l'acide 2-acrylamido-2-méthylpropane sulfonique ou d'au moins un de ses sels.

De manière générale, sauf indication contraire, par « acide 2-acrylamido-2-méthylpropane sulfonique sous forme cristalline hydratée » on désigne la forme acide et/ou la forme salifiée. Il en est de même pour les monomères anioniques qui peuvent désigner les formes acides et/ou salifiées comme, par exemple, pour l'acide acrylique.

Selon un mode préféré de l'invention, le (co)polymère de l'invention est obtenu à partir de la forme saline de l'acide 2-acrylamido-2-méthylpropane sulfonique sous forme cristalline hydratée. L'acide 2-acrylamido-2-méthylpropane sulfonique est donc de préférence partiellement ou totalement salifié avant polymérisation. La forme acide d'un monomère peut être salifiée avant et/ou pendant et/ou après la (co)polymérisation du ou des monomères.

De manière tout à fait surprenante, l'utilisation d'au moins un (co)polymère hydrosoluble préparé à partir de la forme cristalline hydratée de l'acide 2-acrylamido-2-méthylpropane sulfonique ou d'au moins un de ses sels permet le traitement efficace de suspensions de solides. C'est bien l'utilisation de la forme cristalline hydratée de l'acide 2-acrylamido-2-méthylpropane sulfonique, dans la préparation du (co)polymère hydrosoluble, qui confère audit (co)polymère des propriétés particulières, permettant d'améliorer le traitement des particules solides en suspensions.

Par définition, un (co)polymère hydrosoluble est un (co)polymère qui donne une solution aqueuse lorsqu'il est dissous sous agitation à 25°C et avec une concentration de 50 g.L$^{-1}$ dans l'eau.

La forme cristalline hydratée de l'acide 2-acrylamido-2-méthylpropane sulfonique a un diagramme de diffraction des rayons X sur poudre comprenant des pics à 10.58°, 11.2°, 12.65°, 13.66°, 16.28°, 18.45°, 20°, 20.4°, 22.5°, 25.5°, 25.88°, 26.47°, 28.52°, 30.28°, 30.8°, 34.09°, 38.19°, 40.69°, 41.82°, 43.74°, 46.04°degrés 2-thêta. L'incertitude de ces pics est généralement de l'ordre de 0.1°.

La cristallographie aux rayons X, radiocristallographie ou diffractométrie de rayons X est une technique d'analyse qui permet d'étudier la structure de la matière cristalline à l'échelle atomique. Elle s'appuie sur le phénomène physique de diffraction des rayons X. Un diffractomètre ayant une source au cuivre peut être utilisé.

Une poudre formée d'une phase cristalline particulière va toujours donner lieu à des pics de diffraction dans les mêmes directions. Ce diagramme de diffraction forme ainsi une véritable signature de la phase cristalline. Il est donc possible de déterminer la nature de chaque phase cristalline au sein d'un mélange ou d'un produit pur.

Cette signature est spécifique à chaque composé cristallin organique ou inorganique, et se présente sous la forme d'une liste de pics de position en angle 2θ (2-thêta).

Cette technique est utilisée pour caractériser la matière, en particulier les différentes formes cristallines qui peuvent exister pour une même molécule chimique.

La forme cristalline hydratée de l'acide 2-acrylamido-2-méthylpropane sulfonique a un spectre infra-rouge à transformée de Fourier comprenant des pics à 3280cm$^{-1}$, 3126cm$^{-1}$, 1657cm$^{-1}$, 1595cm$^{-1}$, 1453cm$^{-1}$, 1395cm$^{-1}$, 1307cm$^{-1}$, 1205cm$^{-1}$, 1164cm$^{-1}$, 1113cm$^{-1}$, 1041cm$^{-1}$, 968cm$^{-1}$, 885cm$^{-1}$, 815cm$^{-1}$, 794cm$^{-1}$. L'incertitude de ces pics est généralement de l'ordre de 8cm$^{-1}$. De manière avantageuse, il s'agit du spectre solide obtenu conventionnellement dans un sel tel que le KBr.

La spectroscopie infra-rouge à transformée de Fourier est l'analyse des vibrations émises, absorbées ou diffusées par les molécules. Cette technique est sensible aux interactions dites courtes (influence de la maille unitaire sur les liaisons). Dans la majorité des cas, les spectres infra-rouges à transformée de Fourier de différents systèmes cristallins diffèrent de manière significative. Le spectre infra-rouge à transformée de Fourier reflète donc les détails de la structure cristalline d'un composé organique.

De manière générale, et sauf indication contraire, le diagramme de diffraction des rayons X et le spectre infra-rouge sont obtenus à 20°C et à une pression de 1 atmosphère (101 325 Pa).

La forme cristalline hydratée de l'acide 2-acrylamido-2-méthylpropane sulfonique a une énergie minimale d'inflammation supérieure à 400 mJ, préférentiellement supérieure à 500 mJ (1 mJ = 10$^{-3}$ joule).

L'énergie minimale d'inflammation représente l'énergie minimale qui doit être fourni à un composé pour provoquer une inflammation. L'énergie peut être électrique ou thermique. L'énergie minimale d'inflammation est une donnée essentielle pour la prise en compte du risque d'explosion lors de la manipulation du produit (transfert, stockage, réaction, mise en forme...).

L'énergie minimale d'inflammation dépend des propriétés de la poudre (composition) ainsi que de sa structure macromoléculaire (granulométrie, forme cristalline, surface spécifique).

Dans le cadre des solides, cette énergie est l'énergie minimale d'une étincelle électrique susceptible d'enflammer un nuage de poussière. Plus la valeur de l'énergie minimale d'inflammation est élevée, moins le solide présente un risque lors de son utilisation, manipulation, stockage.

La mesure de l'énergie minimale d'inflammation est réalisée selon la norme NF EN 13821.

La forme cristalline hydratée de l'acide 2-acrylamido-2-méthylpropane sulfonique présente 4 phénomènes thermiques avec la technique de calorimétrie différentielle à balayage, à 70°C, 100°C, 150°C et 190°C. L'incertitude relative à l'observation de ces phénomènes est généralement de l'ordre de 10°C, avantageusement 5°C ou moins.

Les phénomènes thermiques sont mesurés par calorimétrie différentielle à balayage (DSC). Cette technique exploite la mesure de la variation de chaleur associée à la dénaturation thermique du composé lorsque celui-ci est chauffé à vitesse constante, par exemple avec une rampe de chauffe de 10°C/minute.

Il est généralement reconnu que le phénomène thermique se présentant à 190°C (+/-10°C) est lié au point de fusion de l'acide 2-acrylamido-2-méthylpropane sulfonique.

Selon l'invention, on a trouvé de manière surprenante que l'utilisation d'un (co)polymère préparé à partir de la forme cristalline hydratée de de l'acide 2-acrylamido-2-méthylpropane sulfonique améliore les performances des traitements de suspensions tels que :

- l'augmentation de la concentration des boues à la sortie d'un épaississeur, ou
- l'étape de déshydratation et les étapes de séchage et de solidification des suspensions lorsque celles-ci sont déchargées sur le sol, ou
- le traitement mécanique des suspensions traitées.

Un autre aspect de l'invention est un procédé pour floculer une suspension de particules solides dans l'eau, comprenant la mise en contact de ladite suspension avec au moins un (co)polymère hydrosoluble préparé à partir de la forme cristalline hydratée de l'acide 2-acrylamido-2-méthylpropane sulfonique ou d'au moins un de ses sels.

Selon un mode particulier de l'invention, le (co)polymère hydrosoluble est obtenu au moins à partir d'acide 2-acrylamido-2-méthylpropane sulfonique dont 50% à 100% est avantageusement sous la forme cristalline hydratée, plus avantageusement 70 à 100%, et encore plus avantageusement 100%.

Sauf indication contraire (quantité d'agent de ramification/réticulation et quantité de particules solides dans la suspension à traiter), les pourcentages sont des pourcentages molaires.

Le (co)polymère hydrosoluble est avantageusement obtenu à partir d'entre 0,1 et 100 mol% d'acide 2-acrylamido-2-méthylpropane sulfonique, préférentiellement entre 2 et 60 mol% d'acide 2-acrylamido-2-méthylpropane sulfonique, encore plus préférentiellement entre 3 et 25 mol% d'acide 2-acrylamido-2-méthylpropane sulfonique ; 50% à 100 % de l'acide 2-acrylamido-2-méthylpropane sulfonique étant avantageusement sous la forme cristalline hydratée, plus avantageusement 70 à 100%, et encore plus avantageusement 100%.

Selon un mode préféré de l'invention, le (co)polymère de l'invention est obtenu à partir de la forme saline de l'acide 2-acrylamido-2-méthylpropane sulfonique sous forme cristalline hydratée. L'acide 2-acrylamido-2-méthylpropane sulfonique est donc de préférence partiellement ou totalement salifié avant polymérisation. Il est de préférence totalement salifié.

La forme saline est avantageusement obtenue à partir d'un composé choisi parmi un hydroxyde de métal alcalin ou alcalino-terreux, un oxyde de métal alcalin ou alcalino-terreux, l'ammoniaque, une amine de formule suivante $NR_1R_2R_3$ ($R_1$, $R_2$ et $R_3$ étant avantageusement des groupements hydrocarbonés, notamment des alkyles) ou un carbonate de métal alcalin ou alcalino-terreux. Un métal alcalin préféré est le sodium.

Le (co)polymère hydrosoluble est, de préférence, obtenu à partir de la forme cristalline hydratée de l'acide 2-acrylamido-2-méthylpropane sulfonique ou d'au moins un de ses sels, et d'au moins un monomère non ionique, et/ou au moins un monomère anionique, et/ou au moins un monomère cationique et/ou au moins un monomère zwittérionique.

Le ou les monomères non ioniques pouvant être utilisés dans le cadre de l'invention peuvent être choisis, notamment, dans le groupe comprenant les monomères vinyliques solubles dans l'eau. Des monomères préférés appartenant à cette classe sont, par exemple, l'acrylamide, le méthacrylamide, le N-isopropylacrylamide, le N,N-diméthylacrylamide, le N,N-diéthylacrylamide et le N-méthylolacrylamide. Egalement, peuvent être utilisés la N-vinylformamide, le N-vinyl acétamide, la N-vinylpyridine et la N-vinylpyrrolidone, le N-vinyl imidazole, le N-vinyl succinimide, l'acryloyl morpholine (ACMO), le chlorure d'acryloyl, le méthacrylate de glycidyle, le méthacrylate de glycéryle, la diacétone acrylamide et l'isoprenol. Un monomère non ionique préféré est l'acrylamide.

Selon un mode de réalisation particulier, le (co)polymère hydrosoluble est avantageusement obtenu à partir d'entre 1 et 99,9 mol% de monomère(s) non-ionique(s), de préférence entre 40 et 95 mol% et plus préférentiellement entre 45 et 90 mol%, par rapport au nombre total de monomères. Dans ce cas, le copolymère est avantageusement obtenu à partir d'entre 0,1 et 99 mol% d'acide 2-acrylamido-2-méthylpropane sulfonique, et plus préférentiellement entre 10 et 55 mol% ; 50% à 100% de l'acide 2-acrylamido-2-méthylpropane sulfonique étant avantageusement sous la forme cristalline hydratée, plus avantageusement 70 à 100%, et encore plus avantageusement 100%.

Le ou les monomères anioniques pouvant être utilisés dans le cadre de l'invention peuvent être choisis dans un large groupe. Ces monomères peuvent présenter des fonctions acryliques, vinyliques, maléiques, fumariques, malonique, itaconique, ou allyliques, et contenir un groupe carboxylate, phosphonate, phosphate, sulfate, sulfonate, ou un autre groupe à charge anionique. Le monomère anionique peut être sous forme acide ou bien sous forme de sel de métal alcalino-terreux, de sel de métal alcalin ou de sel d'ammonium. Des exemples de monomères convenables incluent l'acide acrylique ; l'acide méthacrylique ; l'acide itaconique ; l'acide crotonique ; l'acide maléique ; l'acide fumarique ; l'acide acrylamido undécanoïque ; l'acide 3-acrylamido 3-méthylbutanoïque ; l'anhydride maléique ; les monomères de type acide fort présentant par exemple une fonction de type acide sulfonique ou acide phosphonique tels que l'acide vinylsulfonique, l'acide vinylphosphonique, l'acide allylsulfonique, l'acide méthallylsulfonique, l'acide 2-méthylidenepropane-1,3-disulfonique, le 2-sulfoéthylméthacrylate, le sulfopropylméthacrylate, le sulfopropylacrylate, l'acide allylphosphonique, l'acide styrène sulfonique, l'acide 2-acrylamido-2-méthylpropane disulfonique ; et les sels hydrosolubles de ces monomères comme leurs sels de métaux alcalins, de métaux alcalino-terreux, ou d'ammonium. Dans cette liste, les monomères mentionnés de type acide fort présentant une fonction de type acide sulfonique n'incluent pas la forme cristalline hydratée de l'acide 2-acrylamido-2-méthylpropane sulfonique.

Selon un mode de réalisation particulier, le (co)polymère est avantageusement obtenu à partir d'entre 0,1 et 100 mol% de monomère(s) anionique(s), préférentiellement entre 1 et 99 mol%, plus préférentiellement entre 5 et 70 mol%, et encore plus préférentiellement entre 10 et 50 mol%, par rapport au nombre total de monomères. Dans ce cas, ces pourcentages incluent également le monomère de forme cristalline hydratée de l'acide 2-acrylamido-2-méthylpropane sulfonique selon l'invention.

Le ou les monomères cationiques pouvant être utilisés dans le cadre de l'invention peuvent être choisis, notamment parmi les monomères dérivés des motifs de type acrylamide, acrylique, vinylique, allylique ou maléique, ces monomères possédant une fonction phosphonium ou ammonium quaternaire. On peut citer, en particulier et de façon non limitative, l'acrylate de diméthylaminoéthyle (ADAME) quaternisé, le méthacrylate de diméthylaminoéthyle (MADAME) quaternisé, le chlorure de diméthyldiallylammonium (DADMAC), le chlorure d'acrylamido propyltriméthyl ammonium (APTAC), et le chlorure de méthacrylamido propyltriméthyl ammonium (MAPTAC). L'agent de quaternisation peut être choisi parmi les chlorures d'alkyle, les sulfates de dialkyle ou les halogénures d'alkyle. Préférentiellement, l'agent de quaternisation est choisi parmi le chlorure de méthyle ou le diéthyle sulfate.

Les sels acidifiés sont obtenus par les moyens connus de l'homme de métier, et notamment par protonation. Les sels quaternisés sont également obtenus par les moyens connus de l'homme du métier notamment, par réaction avec le chlorure de benzyle, le chlorure de méthyle (MeCl), les chlorures d'aryle, d'alkyle, ou les dialkylsulfates comme le diméthylsulfate.

Selon un mode de réalisation préféré, le monomère cationique est choisi parmi les sels de diallyldialkyl ammonium comme le chlorure de diallyl diméthyl ammonium (DADMAC) ; les sels acidifiés ou quaternisés de dialkyl- aminoalkylacrylamides ou méthacrylamides, comme par exemple le chlorure de méthacrylamido-propyl triméthyl ammonium (MAPTAC), le chlorure d'acrylamido-propyl triméthyl ammonium (APTAC).

Lorsqu'on utilise un monomère présentant un caractère cationique pour la préparation du (co)polymère hydrosoluble, sa quantité se situe avantageusement dans la plage comprise entre 0,01 et 20 % en mole par rapport à la quantité totale de monomères, plus préférentiellement entre 0.2 et 6 mol%.

Le monomère zwitterionique peut être un dérivé d'un motif de type acrylamide, acrylique, vinylique, allylique ou maléique, ce monomère possédant une fonction amine ou ammonium quaternaire et une fonction acide de type carboxylique (ou carboxylate), sulfonique (ou sulfonate) ou phosphorique (ou phosphate). On peut citer, en particulier et de façon non limitative les dérivés de l'acrylate de diméthylaminoéthyl, tel que le 2-((2-(acryloyloxy)éthyl) diméthylammonio) éthane-1-sulfonate, le 3-((2-(acryloyloxy)éthyl) diméthylammonio) propane-1-sulfonate, le 4-((2-(acryloyloxy)éthyl) diméthylammonio) butane-1-sulfonate, le [2-(acryloyloxy)éthyl] (diméthylammonio) acetate, les dérivés du méthacrylate de diméthylaminoéthyle tel que le 2-((2-(méthacryloyloxy) éthyl) diméthylammonio) éthane-1-sulfonate, le 3-((2-(méthacryloyloxy) éthyl) diméthylammonio) propane-1-sulfonate, le 4-((2-(méthacryloyloxy) éthyl) diméthylammonio) butane-1-sulfonate, le [2-(méthacryloyloxy)éthyl] (diméthylammonio) acétate, les dérivés du diméthylamino propylacrylamide tel que le 2-((3-acrylamidopropyl) diméthylammonio) éthane-1-sulfonate, le 3-((3-acrylamidopropyl) diméthylammonio) propane-1-sulfonate, le 4-((3-acrylamidopropyl) diméthylammonio) butane-1-sulfonate, le [3-(acryloyloxy) propyl] (diméthylammonio) acétate, les dérivés du diméthylamino propyl méthylacrylamide tel que le 2-((3-méthacrylamidopropyl) diméthylammonio) éthane-1-sulfonate, le 3-((3-méthacrylamidopropyl) diméthylammonio) propane-1-sulfonate, le 4-((3-méthacrylamidopropyl) diméthylammonio) butane-1-sulfonate et le [3-(méthacryloyloxy)propyl] (diméthylammonio) acétate.

Lorsqu'on utilise un monomère zwitterionique pour la préparation du (co)polymère hydrosoluble, sa quantité se situe avantageusement dans la plage comprise entre 0,01 et 20 % en mole par rapport à la quantité totale de monomères, plus préférentiellement entre 0.1 et 10 mol%.

Les monomères présentant un caractère hydrophobe peuvent également être utilisés dans la préparation du (co)polymère hydrosoluble utilisé dans le procédé de l'invention. Ils sont de préférence choisis dans le groupe constitué par les esters de l'acide (méth)acrylique présentant une chaîne alkyle, arylalkyle, propoxylée, éthoxylée, ou éthoxylée et propoxylée ; les dérivés du (méth)acrylamide présentant une chaîne alkyle, arylalkyle propoxylée, éthoxylée, éthoxylée et propoxylée, ou dialkyle ; les alkyl aryl sulfonates.

Lorsqu'on utilise un monomère présentant un caractère hydrophobe pour la préparation du (co)polymère hydrosoluble, sa quantité se situe avantageusement dans la plage comprise entre 0,001 et 3 % en mole par rapport à la quantité totale de monomères.

Le (co)polymère hydrosoluble est préférentiellement un (co)polymère anionique à base d'acrylamide et d'acide 2-acrylamido-2-méthylpropane sulfonique ; 50% à 100% de l'acide 2-acrylamido-2-méthylpropane sulfonique étant sous la forme cristalline hydratée et/ou ses sels. Préférentiellement, il s'agit d'un terpolymère d'acrylamide, d'acide acrylique et d'acide 2-acrylamido-2-méthylpropane sulfonique ; 50% à 100% de l'acide 2-acrylamido-2-méthylpropane sulfonique étant sous la forme cristalline hydratée et/ou ses sels. Dans les deux cas, le (co)polymère peut être partiellement ou totalement post hydrolysé, les monomères anioniques pouvant être sous forme acide ou salifiée.

De manière préférée, le (co)polymère hydrosoluble contient uniquement des unités monomériques anioniques et non-ioniques. En d'autres termes, il est préférentiellement obtenu à partir d'au moins un monomère anionique et d'au moins un monomère non-ionique.

Selon l'invention, le (co)polymère hydrosoluble peut avoir une structure linéaire, branché, star (en forme d'étoile), comb (en forme de peigne), dendritique ou bloc. Ces structures peuvent être obtenues par sélection au choix de l'amorceur, de l'agent de transfert, de la technique de polymérisation telle que la polymérisation radicalaire contrôlée dite RAFT (transfert de chaîne réversible par addition-fragmentation, de l'anglais « reversible-addition fragmentation chain transfer »), NMP (polymérisation en présence de nitroxydes, de l'anglais « Nitroxide Mediated Polymerization ») ou ATRP (polymérisation radicalaire par transfert d'atomes, de l'anglais « Atom Transfert Radical Polymerization »), de l'incorporation de monomères structuraux, de la concentration...

Selon l'invention, le (co)polymère hydrosoluble est avantageusement linéaire ou structuré. Par (co)polymère structuré, on désigne un (co)polymère non linéaire qui possède des chaînes latérales de manière à obtenir, lorsque ce (co)polymère est mis en solution dans l'eau, un fort état d'enchevêtrement conduisant à des viscosités à bas gradient très importantes. Le (co)polymère hydrosoluble, selon l'invention n'est généralement pas réticulé.

Le (co)polymère hydrosoluble peut en outre être structuré :

- par au moins un agent de structure, pouvant être choisi dans le groupe comprenant des monomères à insaturation polyéthylénique (ayant au minimum deux fonctions insaturées), comme par exemple les fonctions vinyliques, allyliques, acryliques et époxy et l'on peut citer par exemple le méthylène bis acrylamide (MBA), la triallyamine, ou le chlorure de tétraallylammonium ou 1,2 dihydroxyethylène bis-(N-acrylamide), et/ou

- par des macroamorceurs tels que les polyperoxydes, polyazoïques et les polyagents de transfert tels que les (co)polymères polymercaptants, et les polyols, et/ou

- par des polysaccharides fonctionnalisés

La quantité d'agent de ramification/réticulation dans le mélange de monomères est avantageusement inférieure à 4 % en poids par rapport à la teneur en monomères, plus avantageusement inférieure à 1 %, et encore plus avantageusement inférieure à 0,5 %. Selon un mode de réalisation particulier, elle peut être au moins égale à 0.00001 % en poids par rapport à la teneur en monomères.

Les monomères présentant une fonction fluorescente peuvent également être utilisés dans le cadre de l'invention. Un monomère présentant une fonction fluorescente peut être détecté par n'importe quelle méthode appropriée, par exemple par fluorométrie avec un fluorimètre à longueur d'onde fixe. Généralement, la détection du monomère présentant une fonction fluorescente se fait aux maximas d'excitation et d'émission, qui peuvent être déterminés à l'aide d'un fluorimètre à balayage.

Les monomères présentant une fonction fluorescente sont choisis, par exemple, parmi les monomères comprenant le styrène sulfonate de sodium et le styrène sulfonique.

De manière générale, le (co)polymère ne nécessite pas de développement de procédé de polymérisation particulier. En effet, il peut être obtenu selon toutes les techniques de polymérisation bien connues par l'homme de métier. Il peut notamment s'agir de polymérisation en solution ; polymérisation en gel ; polymérisation par précipitation ; polymérisation en émulsion (aqueuse ou inverse) ; polymérisation en suspension ; polymérisation par extrusion réactive ; polymérisation eau dans eau ; ou de polymérisation micellaire.

La polymérisation est généralement une polymérisation à radicaux libres de préférence par polymérisation en émulsion inverse ou par polymérisation en gel. Par polymérisation par radicaux libres, nous incluons la polymérisation par radicaux libres au moyen d'initiateurs UV, azoïques, redox ou thermiques ainsi que les techniques de polymérisation radicalaire contrôlée (CRP) ou les techniques de polymérisation sur matrice.

Selon un mode particulier de l'invention, le (co)polymère peut être post hydrolysé. La post hydrolyse est la réaction du (co)polymère après polymérisation. Cette étape consiste en la réaction de groupes fonctionnels hydrolysables de monomères avantageusement non ioniques, plus avantageusement les fonctions amide ou ester, avec un agent d'hydrolyse. Cet agent d'hydrolyse peut être une enzyme, une résine échangeuse d'ion, ou un métal alcalin. Préférentiellement, l'agent d'hydrolyse est une base. Durant cette étape de posthydrolyse du (co)polymère, le nombre de fonctions acide carboxylique augmente. En effet, la réaction entre la base et les fonctions amides ou esters présentes dans le (co)polymère produit des groupes carboxylates.

Selon l'invention, le (co)polymère peut se présenter sous forme liquide, gel ou solide lorsque sa préparation inclut une étape de séchage tel que le « spray drying » (séchage par pulvérisation), le séchage sur tambour, le séchage par rayonnement tel que le séchage micro-ondes, ou encore le séchage en lit fluidisé.

Selon un mode de réalisation particulier, le (co)polymère hydrosoluble peut comprendre au moins un groupe à LCST.

Selon les connaissances générales de l'homme du métier, un groupe à LCST correspond à un groupe dont la solubilité dans l'eau pour une concentration déterminée, est modifiée au-delà d'une certaine température et en fonction de la salinité. Il s'agit d'un groupe présentant une température de transition par chauffage définissant son manque d'affinité avec le milieu solvant. Le manque d'affinité avec le solvant se traduit par une opacification ou une perte de transparence qui peut être due à une précipitation, une agrégation, une gélification ou une viscosification du milieu. La température de transition minimale est appelée « LCST » (température critique inférieure de solubilité, de l'anglais « Lower Critical Solution Température »). Pour chaque concentration de groupe à LCST, une température de transition par chauffage est observée. Elle est supérieure à la LCST qui est le point minimum de la courbe. En dessous de cette température, le (co)polymère est soluble dans l'eau, au-dessus de cette température, le (co)polymère perd sa solubilité dans l'eau.

Selon un mode de réalisation particulier, le (co)polymère hydrosoluble peut comprendre au moins un groupe à UCST.

Selon les connaissances générales de l'homme du métier, un groupe à UCST correspond à un groupe dont la solubilité dans l'eau pour une concentration déterminée, est modifiée en dessous d'une certaine température et en fonction de la salinité. Il s'agit d'un groupe présentant une température de transition par refroidissement définissant son manque d'affinité avec le milieu solvant. Le manque d'affinité avec le solvant se traduit par une opacification ou une perte de transparence qui peut être due à une précipitation, une agrégation, une gélification ou une viscosification du milieu. La température de transition maximale est appelée « UCST » (température critique supérieure de solubilité, de l'anglais « Upper Critical Solution Température »). Pour chaque concentration de groupe à UCST, une température de transition par refroidissement est observée. Elle est inférieure à la UCST qui est le point maximum de la courbe. Au-dessus de cette température, le (co)polymère est soluble dans l'eau, en-dessous de cette température, le (co)polymère perd sa solubilité dans l'eau.

Selon l'invention, le (co)polymère hydrosoluble a un poids moléculaire avantageusement élevé. Par « poids moléculaire élevé », on désigne des poids moléculaires d'au moins 0,5 million de g/mol, préférentiellement entre 0,5 et 40 millions g/mol, plus préférentiellement entre 5 et 30 millions g/mol. Le poids moléculaire s'entend en poids moléculaire moyen en poids. Le (co)polymère hydrosoluble peut également avoir un poids moléculaire entre 5 000 et 100 000 g/mol ou entre 100 000 et 500 000 g/mol.

Le poids moléculaire est déterminé par la viscosité intrinsèque du (co)polymère. La viscosité intrinsèque peut être mesurée par des méthodes connues de l'homme du métier et peut être calculée à partir des valeurs de viscosité réduite pour différentes concentrations en (co)polymère par méthode graphique consistant à relever les valeurs de viscosité réduite (axe des ordonnées) sur la concentration (axe des abscisses) et d'extrapoler la courbe jusqu'à concentration nulle. La valeur de viscosité intrinsèque est relevée sur l'axe des ordonnées ou en utilisant la méthode des moindres carrés. Le poids moléculaire peut alors être déterminé par l'équation de Mark-Houwink :

$$[\eta] = K \, M^\alpha$$

$[\eta]$ représente la viscosité intrinsèque du (co)polymère déterminée par la méthode de mesure de viscosité en solution.
K représente une constante empirique.
M représente le poids moléculaire du (co)polymère.
$\alpha$ représente le coefficient de Mark-Houwink.
K et $\alpha$ dépendent du système particulier (co)polymère-solvant.

Comme déjà mentionné, l'invention concerne un procédé de traitement d'une suspension de particules solides dans l'eau, comprenant la mise en contact de ladite suspension avec au moins un (co)polymère hydrosoluble, ledit (co)polymère étant préparé à partir de la forme cristalline hydratée de l'acide 2-acrylamido-2-méthylpropane sulfonique ou d'au moins un de ses sels. Il implique le mélange de ladite suspension avec le (co)polymère hydrosoluble de l'invention.

Un tel traitement peut être effectué dans un épaississeur, qui est une zone de retenue, généralement de la forme d'une section de tube d'un diamètre de plusieurs mètres avec un fond conique dans lequel les particules peuvent sédimenter. Selon un mode de réalisation spécifique, on transporte au moyen d'un tuyau (canalisation) la suspension aqueuse jusque vers un épaississeur, on ajoute le (co)polymère hydrosoluble selon l'invention dans ledit tuyau.

Selon un autre mode de réalisation spécifique, le (co)polymère hydrosoluble selon l'invention est ajouté dans un épaississeur qui contient déjà la suspension à traiter. Dans une opération de traitement minéral typique, les suspensions sont souvent concentrées dans un épaississeur. Cela conduit à obtenir une boue de densité plus élevée qui sort par le bas de l'épaississeur, et un fluide aqueux libéré de la suspension traitée (appelé liqueur) qui sort par surverse, par le haut de l'épaississeur. L'addition du (co)polymère hydrosoluble selon l'invention augmente la concentration de la boue et augmente la clarté de la liqueur.

Selon un autre mode de réalisation spécifique, le (co)polymère hydrosoluble selon l'invention est ajouté à la suspension de particules pendant le transport, au moyen d'un tuyau, de ladite suspension vers une zone de dépôt. De préférence, le (co)polymère hydrosoluble selon l'invention est ajouté dans le tuyau qui transporte ladite suspension jusque vers une zone de dépôt. C'est sur cette zone de dépôt que la suspension traitée est répandue en vue de sa déshydratation et sa solidification. Les zones de dépôt peuvent être non fermées, comme par exemple une étendue de sol non délimitée, ou fermées comme par exemple un bassin, une cellule.

Un exemple de ces traitements pendant le transport de la suspension est l'épandage de la suspension traitée avec le (co)polymère hydrosoluble selon l'invention sur le sol en vue de sa déshydratation et sa solidification puis l'épandage d'une deuxième couche de suspension traitée sur la première couche solidifiée. Un autre exemple est l'épandage continu de la suspension traitée avec le (co)polymère hydrosoluble selon l'invention de telle sorte que la suspension traitée tombe en continu sur la suspension préalablement déchargée dans la zone de dépôt, formant ainsi un amas de matériaux traité dont l'eau s'extrait.

Selon un autre mode de réalisation spécifique, le (co)polymère hydrosoluble selon l'invention est ajouté à la suspension, puis un traitement mécanique est effectué, tel qu'une centrifugation, un pressage ou une filtration.

Le (co)polymère hydrosoluble selon l'invention peut être ajouté simultanément dans différents étages du traitement de la suspension, c'est-à-dire par exemple dans le tuyau (canalisation) transportant la suspension vers un épaississeur et dans la boue sortant de l'épaississeur qui sera conduite soit vers une zone de dépôt, soit vers un appareil de traitement mécanique.

Le (co)polymère hydrosoluble selon l'invention peut être ajouté, à la suspension aqueuse à traiter, sous forme liquide ou sous forme solide. Il peut être ajouté sous forme d'une émulsion, (eau dans huile), d'une suspension polyphasique particulaire aqueuse ou huileuse ou d'une poudre. Le (co)polymère est de préférence ajouté sous la forme d'une solution aqueuse obtenue à partir d'une forme concentrée du polymère comme une poudre, une émulsion eau dans huile ou une suspension polyphasique particulaire aqueuse ou huileuse.

Dans un mode particulier selon l'invention, la suspension polyphasique particulaire aqueuse comprend de préférence :

i. 15 à 60 % en masse d'au moins un (co)polymère hydrosoluble selon l'invention sous forme de particule solide de taille moyenne comprise entre 5 et 500 $\mu$m ;
ii. 15 à 45 % en masse d'au moins un sel de métal alcalin et/ou d'au moins un sel d'un métal alcalino-terreux ;
iii. au moins un agent viscosifiant différent du polymère hydrosoluble ;
iv. au moins 10 % en masse d'eau ; et

ladite suspension ayant une viscosité Brookfield comprise entre 500 et 20000 cps à une température de 20°C, et ladite suspension ayant une masse volumique comprise entre 1,1 et 2 kg.L$^{-1}$.

Dans un mode particulier de l'invention, la suspension polyphasique particulaire huileuse comprend de préférence :

i- 15 à 60 % en masse d'au moins un (co)polymère hydrosoluble selon l'invention sous forme de particule solide de taille moyenne comprise entre 5 et 500 $\mu$m ;
ii- au moins un agent viscosifiant différent du polymère hydrosoluble ;
iii- au moins 10% en masse d'huile ; et

ladite suspension ayant une viscosité Brookfield comprise entre 500 et 20000 cps à une température de 20°C, et ladite suspension ayant une masse volumique comprise entre 0,6 et 1,4 kg.L$^{-1}$.

La viscosité Brookfield est mesurée avec un appareil Brookfield, monté d'un module LV, le module pouvant tourner à une vitesse de 30 tours par minutes par exemple, la mesure étant avantageusement effectuée à 20°C. La masse volumique est mesurée à 20°C, à une pression de 1 atm c'est-à-dire 101 325 Pa.

Lorsque le (co)polymère hydrosoluble selon l'invention est sous forme solide, il peut être partiellement ou totalement dissous dans l'eau à l'aide d'une unité de préparation de (co)polymère comme le Polymer Slicing Unit (Unité de tranchage de polymère - PSU) divulgué dans le document EP 2 203 245.

Selon un autre mode de réalisation spécifique, le (co)polymère hydrosoluble selon l'invention est ajouté à la suspension en combinaison avec au moins un autre polymère, synthétique ou naturel. Ces polymères peuvent être ajoutés simultanément ou séparément (avant ou après l'ajout du (co)polymère hydrosoluble selon l'invention). L'autre polymère peut être hydrosoluble ou gonflable dans l'eau. Il peut s'agir d'un dispersant, d'un coagulant ou d'un floculant.

Selon un autre mode de réalisation spécifique, le (co)polymère hydrosoluble selon l'invention est ajouté à la suspension en combinaison avec un sel tel que des sels de calcium et/ou de magnésium. Le (co)polymère hydrosoluble selon l'invention et le sel peuvent être ajoutés simultanément ou séparément. Les sels peuvent être inorganiques ou organiques. Des sels appropriés incluent le chlorure de calcium, l'acétate de calcium, le sulfate de calcium, le nitrate de calcium, l'hydroxyde de calcium, le carbonate de calcium, le chlorure de magnésium, l'acétate de magnésium, le sulfate de magnésium, le nitrate de magnésium, l'hydroxyde de magnésium, le carbonate de magnésium, le formiate de calcium, le gluconate de calcium, le propionate de calcium, le phosphate tricalcique et le succinate de calcium.

Selon l'invention, la quantité (dosage) de (co)polymère hydrosoluble ajouté dans la suspension aqueuse est comprise entre 50 et 5000 g par tonne de solides secs de la suspension, de préférence entre 250 et 2000 g/t, et plus préférablement entre 500 et 1500 g/t, en fonction de la nature et de la composition des suspensions à traiter.

Selon l'invention, le procédé utilisant le (co)polymère décrit dans l'invention permet de traiter efficacement une suspension de particules solides et plus particulièrement de particules minérales.

Les suspensions de particules solides dans l'eau comprennent tous les types de boues, de résidus ou de matériaux de rebut. Les suspensions proviennent plus particulièrement de l'extraction de minerais et se présentent sous la forme de suspensions de particules minérales. Elles peuvent, par exemple, correspondre à des boues ou des résidus industriels et tous les produits de lavage et de rebut de mines provenant de l'exploitation minière, comme par exemple les mines de charbon, les mines de diamant, les mines de phosphate, les mines de métal (aluminium, platine, fer, or, cuivre, argent, etc....). Les suspensions peuvent également provenir de l'extraction de sable bitumeux, par exemple des boues ou des résidus d'extraction dérivés du traitement de sable bitumineux. Ces suspensions comprennent généralement des particules organiques et/ou minérales, telles que, par exemple, des argiles, des sédiments, du sable, des oxydes métalliques, du pétrole, etc.... mélangées avec de l'eau.

Généralement, les suspensions de particules solides sont concentrées et contiennent entre 5 % et 60 % en poids de solides, de préférence entre 20 et 50 % en poids de solides, par rapport au poids total desdites suspensions.

Le procédé selon l'invention peut aussi être utile pour le traitement de résidus provenant de l'extraction de sable bitumineux : des résidus dits « fins » ou « fines tailings », c'est-à-dire contenant une grande quantité d'argiles, et pour le traitement de résidus fins dits « mûrs », les Mature Fine Tailings (MFT), c'est-à-dire ces mêmes résidus fins après quelques années de sédimentation, et contenant une quantité encore plus importante d'argiles. Le procédé selon l'invention peut également être utilisé pour traiter des résidus dits « frais », c'est-à-dire sortant directement de l'opération de séparation du bitume et du sol dont il est extrait.

Les exemples suivants ne sont donnés qu'à titre d'illustration de l'objet de l'invention, sans la limiter en aucune manière.

**EXEMPLES**

Des polymères hydrosolubles de différentes compositions monomériques sont obtenus par réaction dans un réacteur de 1,5 L équipé d'un agitateur mécanique, d'un thermomètre et d'une arrivée d'azote. Les monomères sont introduits dans le réacteur en présence d'eau distillée. Une quantité appropriée de soude est ajoutée afin de neutraliser exactement 100% des monomères acides (ATBS). La concentration totale des monomères dans le mélange réactionnel est de 25% en poids.

Deux types d'ATBS (acide 2-acrylamido-2-méthylpropane sulfonique) poudre sont utilisés, l'un sous forme cristalline hydratée selon l'invention ; l'autre ne se trouve pas sous forme cristalline hydratée, mais sous forme d'aiguille non hydratée.

Le mélange obtenu est homogénéisé puis refroidi et dégazé sous un flux d'azote. La polymérisation est ensuite initiée à l'aide d'un système redox hypophosphite de sodium et tert-Butyl Hydroperoxyde. Le gel résultant, obtenu après polymérisation est ensuite broyé et séché dans un four de séchage de manière à obtenir une poudre.

Les différents polymères préparés sont tous des polymères hydrosolubles de haut poids moléculaires compris entre 10 et 12 millions de g/mol.

Le polymère A (contre-exemple) est un copolymère d'acrylamide (70 mol%) et d'acide 2-acrylamido-2-méthylpropane sulfonique sous forme <u>non</u> cristalline hydratée (30 mol%).

Le polymère B (exemple selon l'invention) est un copolymère d'acrylamide (70 mol%) et d'acide 2-acrylamido-2-méthylpropane sulfonique sous forme cristalline hydratée selon l'invention (30 mol%).

Les polymères A et B sont dissous dans de l'eau du robinet afin d'obtenir des solutions aqueuses présentant une concentration de 0,4 % en poids de polymère par rapport au poids total de la solution. Les deux solutions sont agitées mécaniquement à 500 t/min jusqu'à la solubilisation complète des polymères et l'obtention de solutions claires et homogènes.

Une série de tests de floculation est effectuée sur un effluent minier provenant d'une mine de charbon, et présentant une teneur en solides de 19.6 % en poids.

Une quantité de chaque solution, correspondant à un dosage en polymère de 280 g de polymère par tonne de matière sèche de l'effluent minier, est ajoutée dans 200 g d'effluent minier puis un mélange complet est effectué manuellement jusqu'à ce qu'on observe une floculation et une libération d'eau optimale.

Le résultat est exprimé grâce à la LNE (Libération Nette de l'Eau) qui correspond à la quantité totale d'eau récupérée 1 heure après le test de floculation moins la quantité d'eau indûment ajoutée lors de l'incorporation de la solution aqueuse polymérique dans la suspension. La même LNE est calculée après 24 heures, ce qui permet d'avoir un bon aperçu de la libération maximale d'eau.

La LNE avec le polymère A est de 68 mL contre 84 mL avec le polymère B. La LNE après 24h avec le polymère A est de 72 mL contre 89 mL avec le polymère B. L'eau libérée lors de la floculation avec le polymère B est plus claire que celle libérée lors de la floculation avec le polymère A.

Les résultats de cette expérience démontrent bien que l'utilisation d'acide 2-acrylamido-2-méthylpropane sulfonique sous forme cristalline hydratée selon l'invention permet d'obtenir un polymère plus efficace pour floculer un effluent minier provenant d'une mine de charbon.

Une autre série de test est effectuée sur une boue rouge provenant d'un procédé Bayer, et présentant une teneur en solides de 24.5 % en poids.

Le polymère C (contre-exemple) est un copolymère d'acrylamide (35 mol%) et d'acide 2-acrylamido-2-méthylpropane sulfonique sous forme non cristalline hydratée (65 mol%). Le polymère D (exemple selon l'invention) est un copolymère d'acrylamide (35 mol%) et d'acide 2-acrylamido-2-méthylpropane sulfonique sous forme cristalline hydratée selon l'invention (65 mol%).

Le même protocole de test que celui utilisé sur l'effluent minier provenant d'une mine de charbon est appliqué, à la différence près que la quantité de polymère ajoutée est ici de 740 g de polymère par tonne de matière sèche de boue rouge.

La LNE avec le polymère C est de 42 mL contre 53 mL avec le polymère D. La LNE après 24h avec le polymère C est de 45 mL contre 59 mL avec le polymère D. L'eau libérée lors de la floculation avec le polymère D est plus claire que celle libérée lors de la floculation avec le polymère C.

Les résultats de cette expérience démontrent bien que l'utilisation d'acide 2-acrylamido-2-méthylpropane sulfonique sous forme cristalline hydratée selon l'invention permet d'obtenir un polymère plus efficace pour floculer une boue rouge provenant d'un procédé Bayer.

**Revendications**

1. Procédé de traitement d'une suspension de particules solides dans l'eau, comprenant la mise en contact de ladite suspension avec au moins un (co)polymère hydrosoluble préparé à partir d'acide 2-acrylamido-2-méthylpropane sulfonique ou d'au moins un de ses sels,
l'acide 2-acrylamido-2-méthylpropane sulfonique étant une forme cristalline hydratée de l'acide 2-acrylamido-2-méthylpropane sulfonique ayant un diagramme de diffraction des rayons X sur poudre comprenant des pics à 10.58°, 11.2°, 12.65°, 13.66°, 16.28°, 18.45°, 20°, 20.4°, 22.5°, 25.5°, 25.88°, 26.47°, 28.52°, 30.28°, 30.8°, 34.09°, 38.19°, 40.69°, 41.82°, 43.74°, 46.04° degrés 2-thêta.

2. Procédé selon la revendication 1, *caractérisé* **en ce que** le (co)polymère hydrosoluble est obtenu au moins à partir d'acide 2-acrylamido-2-méthylpropane sulfonique, 50% à 100% de l'acide 2-acrylamido-2-méthylpropane sulfonique étant sous la forme cristalline hydratée.

3. Procédé selon la revendication 1 ou 2, *caractérisé* **en ce que** l'acide 2-acrylamido-2-méthylpropane sulfonique sous forme cristalline hydratée est partiellement ou totalement salifié avant polymérisation.

4. Procédé selon l'une des revendications 1 à 3, *caractérisé* **en ce que** le (co)polymère hydrosoluble est obtenu à partir de la forme cristalline hydratée de l'acide 2-acrylamido-2-méthylpropane sulfonique ou d'au moins un de ses sels, et d'au moins un monomère non ionique, et/ou au moins un monomère anionique, et/ou au moins un monomère cationique, et/ou au moins un monomère zwittérionique.

5. Procédé selon la revendication 4, *caractérisé* **en ce que** le au moins un monomère non ionique est choisi dans le groupe comprenant l'acrylamide, le méthacrylamide, le N-isopropylacrylamide, le N,N-diméthylacrylamide, le N,N-diéthylacrylamide et le N-méthylolacrylamide, la N-vinylformamide, le N-vinyl acétamide, la N-vinylpyridine et la N-vinylpyrrolidone, le N-vinyl imidazole, le N-vinyl succinimide, l'acryloyl morpholine (ACMO), le chlorure d'acryloyl, le méthacrylate de glycidyle, le méthacrylate de glycéryle, la diacétone acrylamide et l'isoprenol.

6. Procédé selon l'une des revendications 1 à 5, *caractérisé* **en ce que** le (co)polymère hydrosoluble est obtenu à partir d'entre 1 et 99,9 mol% de monomère(s) non-ionique(s) par rapport au nombre total de monomères, et comprend à partir d'entre 0,1 et 99 mol% d'acide 2-acrylamido-2-méthylpropane sulfonique, 50% à 100% de l'acide 2-acrylamido-2-méthylpropane sulfonique étant sous la forme cristalline hydratée.

7. Procédé selon la revendication 4, *caractérisé* **en ce que** le monomère anionique est choisi parmi l'acide acrylique, l'acide méthacrylique, l'acide itaconique, l'acide crotonique, l'acide maléique, l'acide fumarique, l'acide acrylamido undécanoïque, l'acide 3-acrylamido 3-méthylbutanoïque, l'anhydride maléique ; l'acide vinylsulfonique, l'acide vinylphosphonique, l'acide allylsulfonique, l'acide méthallylsulfonique, l'acide 2-méthylidenepropane-1,3-disulfonique, le 2-sulfoéthylméthacrylate, le sulfopropylméthacrylate, le sulfopropylacrylate, l'acide allylphosphonique, l'acide styrène sulfonique, l'acide 2-acrylamido-2-méthylpropane disulfonique ; et les sels hydrosolubles de ces monomères comme leurs sels de métaux alcalins, de métaux alcalino-terreux, ou d'ammonium.

8. Procédé selon l'une des revendications 1 à 7, *caractérisé* **en ce que** le (co)polymère hydrosoluble est un copolymère anionique à base d'acrylamide et d'acide 2-acrylamido-2-méthylpropane sulfonique, 50% à 100% de l'acide 2-acrylamido-2-méthylpropane sulfonique étant sous la forme cristalline hydratée et/ou ses sels, ou un terpolymère d'acrylamide, d'acide acrylique et d'acide 2-acrylamido-2-méthylpropane sulfonique, 50% à 100% de l'acide 2-acrylamido-2-méthylpropane sulfonique étant sous la forme cristalline hydratée et/ou ses sels.

9. Procédé selon l'une des revendications 1 à 8, *caractérisé* **en ce que** le (co)polymère hydrosoluble est obtenu à partir d'une quantité de monomères anioniques comprise entre 5 et 70 mol%.

10. Procédé selon l'une des revendications 1 à 9, *caractérisé* **en ce que** le (co)polymère hydrosoluble a un poids moléculaire moyen en poids compris entre 0,5 et 40 millions g/mol.

11. Procédé selon l'une des revendications 1 à 10, *caractérisé* **en ce que** la quantité de (co)polymère hydrosoluble ajouté dans la suspension aqueuse est comprise entre 50 et 5000 g par tonne de solides secs de la suspension.

12. Procédé selon l'une des revendications 1 à 11, *caractérisé* **en ce que** la suspension aqueuse de particules solides provient de l'extraction de minerais et consiste en une suspension de particules minérales.

13. Procédé selon l'une des revendications 1 à 12, *caractérisé* **en ce que** la suspension de particules solides contient entre 5 % et 60 % en poids de solides.

14. Procédé selon l'une des revendications 1 à 13, *caractérisé* **en ce qu'**on transporte au moyen d'un tuyau la suspension aqueuse jusque vers une zone de dépôt et **en ce qu'**on ajoute le (co)polymère hydrosoluble dans ledit tuyau.

15. Procédé pour floculer une suspension de particules solides dans l'eau, comprenant la mise en contact de ladite suspension avec au moins un (co)polymère hydrosoluble préparé au moins à partir de la forme cristalline hydratée de l'acide 2-acrylamido-2-méthylpropane sulfonique ou d'au moins un de ses sels, la forme cristalline hydratée de l'acide 2-acrylamido-2-méthylpropane sulfonique ayant un diagramme de diffraction des rayons X sur poudre comprenant des pics à 10.58°, 11.2°, 12.65°, 13.66°, 16.28°, 18.45°, 20°, 20.4°, 22.5°, 25.5°, 25.88°, 26.47°, 28.52°, 30.28°, 30.8°, 34.09°, 38.19°, 40.69°, 41.82°, 43.74°, 46.04° degrés 2-thêta.

**Patentansprüche**

1. Verfahren zur Behandlung einer Suspension fester Partikel in Wasser, bei dem diese Suspension mit mindestens einem wasserlöslichen (Co)polymer in Kontakt gebracht wird, das aus 2-Acrylamido-2-methylpropansulfonsäure oder mindestens einem ihrer Salze hergestellt wird, bei der 2-Acrylamido-2-methylpropansulfonsäure handelt es sich um eine hydratisierte kristalline Form der 2-Acrylamido-2-methylpropansulfonsäure mit einem Röntgen-Pulverdiffraktogramm das Spitzen bei 10.58°, 11.2°, 12.65°, 10 13.66°, 16.28°, 18.45°, 20°, 20.4°, 22.5°, 25.5°, 25.88°, 26.47°, 28.52°, 30.28°, 30.8°, 34.09°, 38.19°, 40.69°, 41.82°, 43.74°, 46.04° Grad 2-theta aufweist.

2. Verfahren nach Anspruch 1, *dadurch gekennzeichnet, dass* das wasserlösliche (Co)Polymer hergestellt wird aus mindestens 2-Acrylamido-2-methylpropansulfonsäure, 50 % bis 100% der 2-Acrylamido-2-methylpropansulfonsäure haben dabei die hydratisierte kristalline Form.

3. Verfahren nach einem der Ansprüche 1 - 2, *dadurch gekennzeichnet, dass* die 2-Acrylamido-2-methylpropansulfonsäure in der hydratisierten kristallinen Form vor der Polymerisation teilweise oder vollständig in Salz überführt wurde.

4. Verfahren nach einem der Ansprüche 1 - 2, *dadurch gekennzeichnet, dass* das wasserlösliche (Co)Polymer hergestellt wird aus der hydratisierten kristallinen Form der 2-Acrylamido-2-methylpropansulfonsäure oder mindestens einem ihrer Salze, und mindestens einem nicht ionischen Monomer, und/oder mindestens einem anionischen Monomer und/oder mindestens einem kationischen Monomer und/oder mindestens einem zwitterionischen Monomer.

5. Verfahren nach Anspruch 4, *dadurch gekennzeichnet, dass* das mindestens eine nichtionische Element ausgewählt wird aus der Gruppe zu der Acrylamid, Methacrylamid, N-Isopropylacrylamid, N,N-Dimethylacrylamid, N,N-Diethylacrylamid und N-Methylolacrylamid, N-Vinylformamid, N-Vinylacetamid, N-Vinylpyridin und N-Vinylpyrrolidon, N-Vinylimidazol, N-Vinylsuccinimid, Acryloylmorpholin (ACMO), Acryloylchlorid, Glycidylemethacrylat, Glycerylemethacrylat°, Diacetonacrylamid und Isoprenol gehören.

6. Verfahren nach einem der Ansprüche 1 bis 5, *dadurch gekennzeichnet, dass* das wasserlösliche (Co)polymer hergestellt wird aus zwischen 1 und 99,9 mol% nichtionischen Monomere(n), bezogen auf die Gesamtzahl der Monomere und ab zwischen 0,1 und 99 mol% 2-Acrylamido-2-methylpropansulfonsäure, 50% bis 100% 2-Acrylamido-2-methylpropansulfonsäure haben dabei die hydratisierte kristalline Form.

7. Verfahren nach Anspruch 4, *dadurch gekennzeichnet, dass* das anionische Monomer ausgewählt wird aus Acrylsäure, Methacrylsäure, Itaconsäure, Crotonsäure, Maleinsäure, Fumarsäure, Acrylamido- Undecan- Säure, 3-Acrylamido- 3- Methylbutansäure, Maleinanhydrid; Vinylsulfonsäure, Vinylphosphonsäure, Allylsulfonsäure, Methallylsulfonsäure, 2-Methylidenpropan- 1,3 - disulfonsäure, 2- Sulfoethylmethacrylat, Sulfopropylmethacrylat, Sulfopropylacrylat, Allylphosphonsäure, Styrolsulfonsäure, 2- Acrylamido- 2- methylpropandisulfonsäure; und die wasserlöslichen Salze dieser Monomere, sowie ihre Alkalimetallsalze, Erdalkalimetallsalze oder Ammoniumsalze

8. Verfahren nach einem der Ansprüche 1 bis 7, *dadurch gekennzeichnet, dass* das wasserlösliche (Co)polymer ein anionisches Copolymer auf Basis von Acrylamid und 2- Acrylamido- 2- methylpropansulfonsäure ist, 50% bis 100% 2- Acrylamido-2- methylpropansulfonsäure haben dabei die hydratisierte kristalline Form und/ oder ihrer Salze, oder eines Acrylamid- Terpolymer, aus Acrylsäure und 2- Acrylamido-2- mehylpropansulfonsäure, 50% bis 100 % der Acrylamido- 2-mehylpropansulfonsäure haben dabei die hydratisierte kristalline Form und/ oder sind ihre Salze.

9. Verfahren nach einem der Ansprüche 1 bis 8, *durch gekennzeichnet, dass* das wasserlösliche (Co)polymer aus einer Menge an anionischen Monomeren hergestellt wird, die zwischen 5 und 70 mol% liegt.

10. Verfahren nach einem der Ansprüche 1 bis 9, *durch gekennzeichnet, dass* das wasserlösliche (Co)polymer ein durchschnittliches Molekulargewicht zwischen 0,5 et 40 Millionen g/mol aufweist.

11. Verfahren nach einem der Ansprüche 1 bis 1°, *durch gekennzeichnet, dass* die zu der wässrigen Suspension hinzugegebene Menge an wasserlöslichem (Co)polymer zwischen 50 und 5000 g pro Tonne trockener Feststoffe der Suspension beträgt.

12. Verfahren nach einem der Ansprüche 1 bis 11, *durch gekennzeichnet, dass* die wässrige Suspension fester Partikel aus der Extraktion von Mineralstoffen stammt und aus einer Suspension von Mineralpartikeln besteht.

13. Verfahren nach einem der Ansprüche 1 bis 12, *durch gekennzeichnet, dass* die Suspension fester Partikel zwischen 5 und 60 Gewichts- % Feststoffe enthält.

14. Verfahren nach einem der Ansprüche 1 bis 13, *durch gekennzeichnet, dass* mittels eines Schlauchs die wässrige Suspension zu einer Absetzzone transportiert wird und dass das wasserlösliche (Co)polymer in diesen Schlauch hinzugegeben wird.

15. Verfahren zum Ausflocken einer Suspension fester Partikel in Wasser, dieses beinhaltet, diese Suspension mit mindestens einem wasserlöslichen (Co)Polymer in Kontakt zu bringen, das hergestellt wird aus der hydratisierten kristallinen Form von 2- Acrylamido- 2- methylpropansulfonsäure und/ oder mindestens einem der Salze, die kristalline Form von 2- Acrylamido- 2- methylpropansulfonsäure hat dabei ein Röntgen- Pulverdiffraktogramm, das Spitzen bei 10.58°, 11.2°, 12.65°, 13.66°, 16.28°, 18.45°, 20°, 20.4°, 22.5°, 25.5°, 25.88°, 26.47°, 28.52°, 30.28°, 30.8°, 34.09°, 38.19°, 40.69°, 41.82°, 43.74°, 46.04° Grad 2- theta aufweist.

## Claims

1. A method for the treatment of a suspension of solid particles in water, comprising the placing of said suspension in contact with at least one water-soluble (co)polymer made from 2-acrylamido-2-methylpropane sulfonic acid or of at least one of the salts thereof,
the 2-acrylamido-2-methylpropane sulfonic acid being a hydrated crystalline form of 2-acrylamido-2-methylpropane sulfonic acid having a 2-theta powder X-ray diffraction diagram comprising peaks at 10.58°, 11.2°, 12.65°, 13.66°, 16.28°, 18.45°, 20°, 20.4°, 22.5°, 25.5°, 25.88°, 26.47°, 28.52°, 30.28°, 30.8°, 34.09°, 38.19°, 40.69°, 41.82°, 43.74°, 46.04° degrees.

2. The method according to claim 1, *characterized in that* the water-soluble (co)polymer is at the least made from 2-acrylamido-2-methylpropane sulfonic acid, 50% to 100% 2-acrylamido-2-methylpropane sulfonic being in hydrated crystalline form.

3. The method according to claim 1 or 2, *characterized in that* the 2-acrylamido-2-methylpropane sulfonic acid in hydrated crystalline form is partially or totally salified before polymerization.

4. The method according to claims 1 to 3, **characterized in that** the water-soluble (co)polymer is made from the hydrated crystalline form of 2-acrylamido-2-methylpropane sulfonic acid or of at least one of the salts thereof, and from at least one nonionic monomer, and/or at least one anionic monomer, and/or at least one cationic monomer and/or at least one zwitterionic monomer.

5. The method according to claim 4, *characterized in that* the at least one nonionic monomer is chosen from the group comprising acrylamide, methacrylamide, N-isopropylacrylamide, N,N-dimethylacrylamide, N,N-diethylacrylamide and N-methylolacrylamide, N-vinylformamide, N-vinyl acetamide, N-vinylpyridine, N-vinylpyrrolidone, N-vinyl imidazole, N-vinyl succinimide, acryloyl morpholine (ACMO), acryloyl chloride, glycidyl methacrylate, glyceryl methacrylate, diacetone acrylamide and isoprenol.

6. The method according to one of claims 1 to 5, *characterized* in that the water-soluble (co)polymer is made from between 1 and 99.9 mol% of nonionic monomer(s) in relation to the total number of monomers and comprises from 0.1 to 99 mol% 2-acrylamido-2-methylpropane sulfonic acid, 50% to 100% of the 2-acrylamido-2-methylpropane sulfonic acid being in hydrated crystalline form.

7. The method according to claim 4, *characterized* in that the anionic monomer is chosen from acrylic acid, methacrylic acid, itaconic acid, crotonic acid, maleic acid, fumaric acid, acrylamido undecanoic acid, 3-acrylamido 3-methylbutanoic acid, maleic anhydride, vinylsulfonic acid, vinylphosphonic acid, allylsulfonic acid, methallylsulfonic acid, 2-methylidenepropane-1,3-disulfonic acid, 2-sulfoethylmethacrylate, sulfopropylmethacrylate, sulfopropylacrylate, allylphosphonic acid, styrene sulfonic acid, 2-acrylamido-2-methyl propane disulfonic acid; and water-soluble salts of these monomers such as the alkali metal, alkaline earth metal, or ammonium salts thereof.

8. The method according to claims 1 to 7, *characterized* in that the water-soluble (co)polymer is an anionic (co)polymer based on acrylamide and 2-acrylamido-2-methylpropane sulfonic acid, 50% to 100% of the 2-acrylamido-2-methylpropane sulfonic acid being in the hydrated crystalline form and/or the salts thereof, or a terpolymer of acrylamide, of acrylic acid and of 2-acrylamido-2-methylpropane sulfonic acid, 50% to 100% of the 2-acrylamido-2-methylpropane sulfonic acid being in the hydrated crystalline form and/or the salts thereof.

9. The method according to one of claims 1 to 8, *characterized* in that the water-soluble (co)polymer is made from a quantity of anionic monomers of between 5 and 70 mol%.

10. The method according to one of claims 1 to 9, *characterized* in that the water-soluble (co)polymer has aa average molecular weight in weight of between 0.5 and 40 million g/mol.

11. The method according to one of claims 1 to 10, *characterized* in that the quantity of water-soluble (co)polymer added to the aqueous suspension is between 50 and 5,000 g per metric ton of dry solids of suspension.

12. The method according to one of claims 1 to 11, *characterized* in that the aqueous suspension of solid particles results from ore extraction and consists of a suspension of mineral particles.

13. The method according to claims 1 to 12, *characterized* in that the suspension of solid particles contains between 5% and 60% by weight of solids.

14. The method according to one of claims 1 to 13, *characterized* in that the aqueous suspension is transported by means of a pipe to a depositing area and **in that** the water-soluble (co)polymer is added within said pipe.

15. A method for the flocculation of a suspension of solid particles in water, comprising the placing of said suspension in contact with at least one water-soluble (co)polymer made from the hydrated crystalline form of 2-acrylamido-2-methylpropane sulfonic acid or of at least one of the salts thereof,
the hydrated crystalline form of 2-acrylamido-2-methylpropane sulfonic acid having a 2-theta powder X-ray diffraction diagram comprising peaks at 10.58°, 11.2°, 12.65°, 13.66°, 16.28°, 18.45°, 20°, 20.4°, 22.5°, 25.5°, 25.88°, 26.47°, 28.52°, 30.28°, 30.8°, 34.09°, 38.19°, 40.69°, 41.82°, 43.74°, 46.04° degrees.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4347140 A
- CA 1273888
- WO 9605146 A
- CA 2407869
- CA 1515581
- CA 2682542
- WO 2016128638 A
- EP 2203245 A